# EUROPÄISCHE PATENTANMELDUNG

(11) **EP 2 363 103 A1**
(43) Veröffentlichungstag der Anmeldung: **07.09.2011**
(21) Anmeldenummer: 11001766.2
(22) Anmeldetag: 03.03.2011
(51) Int. Cl.: A61F 9/007, A61M 1/00

(54) **Steuer-/Versorgungseinheit zum Betreiben eines medizinischen Handgeräts**

(30) Priorität: 04.03.2010 DE 102010010336
(71) Anmelder: Geuder AG, 69126 Heidelberg (DE)
(72) Erfinder: Geuder, Volker, 69126 Heidelberg (DE); Frauenfeld, Dieter, 69121 Heidelberg (DE); Wolschendorf, Marc, 69121 Heidelberg (DE)
(74) Vertreter: Ullrich & Naumann

(57) **Zusammenfassung**

Eine Steuer-/Versorgungseinheit zum Betreiben mindestens eines medizinischen Handgeräts (2), Handstücks oder Handgriffs, insbesondere eines chirurgischen Handgeräts (2) zur Durchführung ophthalmologischer Operationen, wobei eine Versorgungsfunktion zum Versorgen eines Handgeräts mit einer Flüssigkeit aus einem Vorratsbehälter und eine Aspirationsfunktion zum Absaugen der Flüssigkeit über das Handgerät (2) in eine Aspirationskammer vorgesehen ist, in die die Flüssigkeit vom Handgerät (2) über einen Schlauch förderbar ist, ist dadurch gekennzeichnet, dass eine Sensorik (8) zum Überwachen des Füllstands bzw. der Füllstandsänderung in dem Vorratsbehälter und somit des Zuflusses pro Zeiteinheit zum Handgerät vorgesehen ist und dass über die ermittelte Füllstandsänderung im Vorratsbehälter bzw. über den ermittelten Zufluss zum Handgerät die Pumpenleistung bzw. Saugleistung zum Absaugen der Flüssigkeit in die Aspirationskammer (6) regelbar ist.

## Beschreibung

Die vorliegende Erfindung betrifft eine Steuer-/Versorgungseinheit zum Betreiben mindestens eines medizinischen Handgeräts, Handstücks oder Handgriffs, insbesondere eines chirurgischen Handgeräts zur Durchführung ophthalmologischer Operationen, wobei eine Versorgungsfunktion zum Versorgen eines Handgeräts mit einer Flüssigkeit aus einem Vorratsbehälter und eine Aspirationsfunktion zum Absaugen der Flüssigkeit über das Handgerät in eine Aspirationskammer vorgesehen ist, in die die Flüssigkeit vom Handgerät über einen Schlauch förderbar ist.

Steuer-Nersorgungseinheiten der gattungsbildenden Art sind seit Jahren aus der Praxis bekannt. Sie sind als bauliche Einheit zu verstehen und versorgen beispielsweise in der Ophthalmologie die vom Operateur benötigten Handgeräte mit Druckluft, pulsierender Druckluft, Unterdruck, Licht, Strom, Flüssigkeit, etc. und dienen ebenso zum Erzeugen eines Unterdrucks zum Absaugen von Flüssigkeit, beispielsweise im Rahmen der Entfernung des Linsenkörpers nach dessen Zertrümmerung, wobei dabei die Linsentrümmer gemeinsam mit einer Spülflüssigkeit abgesaugt wird.

Bei den durch die Steuer-Nersorgungseinheit zu betreibenden medizinischen Handgeräten kann es sich um Handgeräte unterschiedlichster Art handeln, beispielsweise um ein chirurgisches Instrument, wie es aus der EP 1 844 705 B1 bekannt ist. Dort geht es im Konkreten um ein zur Beleuchtung des Inneren des Auges dienendes Instrument, welches von einer Steuer-Nersorgungseinheit her über einen Lichtleiter als Versorgungsleitung mit Licht versorgt wird. Ein solches Instrument lässt sich beispielsweise zur Intraokularbeleuchtung verwenden.

Des Weiteren kann es sich bei dem an die Steuer-Nersorgungseinheit anzuschließenden Handgerät um ein augenchirurgisches Instrument zum Zertrümmern von Linsen und zum Absaugen von Linsentrümmern aus dem Auge handeln, wie es aus der DE 40 08 594 C2 bekannt ist. In diesem Falle wird das Handgerät von der Steuer-Nersorgungseinheit her mit pulsierender Druckluft und Spülflüssigkeit versorgt, wobei die zertrümmerten Linsenteile per Unterdruck abgesaugt werden. Die Schlauchverbindung zwischen dem Handgerät und der Steuer-Nersorgungseinheit dient dabei sowohl zur Versorgung als auch zur Entsorgung.

An dieser Stelle sei angemerkt, das es für die erfindungsgemäße Lehre keine Rolle spielt, welche sonstigen Funktionen die Steuer-/Versorgungseinheit umfasst bzw. dem Operateur anbietet, zumal es hier einerseits um die Versorgung des Handgeräts und somit des Auges mit einer Flüssigkeit und andererseits um das Absaugen, d.h. um die Aspiration von Flüssigkeit durch eine entsprechende Steuer-Nersorgungseinheit geht. Ein geeignetes Handgerät wird durch die erfindungsgemäße Steuer-Nersorgungseinheit betrieben bzw. gesteuert, wobei zum Absaugen der Flüssigkeit ein Unterdruck im Saugsystem erzeugt wird. Diesen Unterdruck gilt es bei geringstmöglicher Pumpenlast zu steuern bzw. zu regeln.

Aus der Praxis ist es bereits bekannt, Aspirationsflüssigkeit in eine entsprechende Einheit zu saugen bzw. zu pumpen und diese von dort aus zu entsorgen. Die Füllstandsüberwachung kann dabei kapazitiv erfolgen, wobei es dazu erforderlich ist, die Elektroden einer kapazitiven Sensorik innerhalb des die Aspirationsflüssigkeit aufnehmenden Behältnisses anzuordnen. Dies ist aufwendig und erfordert eine umständliche Handhabung. Außerdem ist der Zugriff auf die die Aspirationsflüssigkeit aufnehmende Aspirationskammer erschwert. Gleiches gilt für die Reinigung.

Im Stand der Technik ist es außerdem nachteilig, dass die den Unterdruck generierende Pumpe meist unter Volllast läuft und die Leitung beim Erfordernis eines geringeren Unterdrucks belüftet wird. Ebenso wird der Unterdruck über ein Ventil zwischen einer Unterdruckkammer und dem Vorratsbehälter gedrosselt, wenngleich die Pumpenleistung weiterhin aufrechterhalten wird. Eine unter Volllast laufende Pumpe verursacht ganz erhebliche Geräusche, die vom Operateur in unangenehmer Weise wahrgenommen werden.

Der vorliegenden Erfindung liegt daher die Aufgabe zugrunde, die gattungsbildende Steuer-Nersorgungseinheit derart auszugestalten und weiterzubilden, dass das Absaugen, Überwachen und Entsorgen der Aspirationsflüssigkeit auf einfache Weise möglich ist. Die dazu erforderliche Vorrichtung soll einfach in der Konstruktion und leicht zu handhaben sein. Insbesondere soll der zur Aspiration erforderliche Unterdruck auf sinnvolle Weise derart geregelt werden, dass die Pumpe nur dann unter Volllast läuft, wenn dies auch tatsächlich erforderlich ist.

Die voranstehende Aufgabe ist durch die Merkmale des Patentanspruchs 1 gelöst. Danach ist die erfindungsgemäße Steuer-/Versorgungseinheit dadurch gekennzeichnet, dass eine Sensorik zum Überwachen des Füllstands bzw. der Füllstandsänderung in dem Vorratsbehälter und somit des Zuflusses pro Zeiteinheit zum Handgerät vorgesehen ist und dass über die ermittelte Füllstandsänderung im Vorratsbehälter bzw. über den ermittelten Zufluss zum Handgerät die Pumpenleistung bzw. Saugleistung zum Absaugen der Flüssigkeit in die Aspirationskammer regelbar ist.

Erfindungsgemäß ist erkannt worden, dass eine Sensorik zum Überwachen des Füllstands bzw. der Füllstandsänderung in dem Vorratsbehälter vorgesehen ist, um nämlich den vom Vorratsbehälter zum Handgerät und somit in das Auge generierten Zufluss pro Zeiteinheit exakt festzustellen. Die Regelung basiert auf der Logik, dass maximal diejenige Menge an Flüssigkeit abgesaugt bzw. aspiriert werden kann, die dem Handgerät aus dem Vorratsbehälter zugeführt worden ist. Entsprechend wird über die ermittelte Füllstandsänderung im Vorratsbehälter bzw. über den ermittelten Zufluss an Flüssigkeit zum Handgerät die Pumpenleistung bzw. Saugleistung zum Absaugen der Flüssigkeit in die Aspirationskammer geregelt, so dass es aufgrund dieser Regelung möglich ist, die Pumpe mit geringstmöglicher Last zu fahren.

Die Füllstandsänderung kann über Gewichtssensoren, insbesondere über Wegezellen erfolgen, die der Steuer-/Versorgungseinheit oder einem Stativ zum Aufhängen des Vorratsbehälters zugeordnet sind. Ebenso ist es denkbar und von besonderem Vorteil, wenn die Füllstandsänderung über kapazitive oder lichtoptische Füllstandssensoren ermittelbar ist. Auf diese wird später noch Bezug genommen werden.

In weiter vorteilhafter Weise wird die den Unterdruck zur Aspiration generierende Pumpe zu Beginn eines Absaugvorgangs mit hoher Spannung angesteuert und danach mit reduzierter Spannung, vorzugsweise gepulst, in Abhängigkeit von der Füllstandsänderung im Vorratsbehälter weiterbetrieben, und zwar unter Zugrundelegung der ermittelten Daten entsprechend den voranstehenden Ausführungen. Die Füllstandsänderung im Vorratsbehälter wird als Stellgröße für die Regelung der Pumpenleistung genutzt.

In weiter vorteilhafter Weise ist eine Sensorik zum Überwachen des Füllstands in der Aspirationskammer vorgesehen, die bei Erreichen eines vorgegebenen Füllstands über ein entsprechendes Signal das Abpumpen der in der Aspirationskammer befindlichen Flüssigkeit in ein separates Behältnis aktiviert.

Insoweit ist erkannt worden, dass es von Vorteil ist, wenn der Füllstand in der Aspirationskammer überwacht wird, und zwar nicht nur unter dem Aspekt, bei Erreichen eines vorgegebenen Füllstands die Aspirationskammer - manuell - zu entleeren. Vielmehr dient ein bei Erreichen eines vorgegebenen Füllstands durch die Sensorik erzeugtes Signal zum automatischen Abpumpen der in der Aspirationskammer befindlichen Flüssigkeit, genauer gesagt zum Abpumpen der Flüssigkeit in ein separates Behältnis. Mit anderen Worten wird mit Erreichen des vorgegebenen Füllstands der Abpumpvorgang aktiviert und kann der Abpumpungsvorgang bei vollständiger Entleerung der Aspirationskammer oder bei Erreichen einer unteren Füllstandsmarke beendet werden.

In vorteilhafter Weise wird zum Absaugen der Flüssigkeit Unterdruck innerhalb der Kassette erzeugt, nämlich in einer besonderen Unterdruckkammer. Dies bedeutet, dass die aus der Einheit entnehmbare bzw. austauschbare Kassette sowohl eine Aspirationskammer zur Aufnahme der abgesaugten Flüssigkeit als auch eine Unterdruckkammer zum Bereitstellen des Unterdrucks aufweist, wobei zwischen den beiden Kammern eine Trennung dahingehend geschaffen ist, dass die abgesaugte Flüssigkeit nicht in die Unterdruckkammer und vor allem nicht in den Bereich der den Unterdruck generierenden Pumpe gelangen kann. Der Unterdruck kann auf unterschiedliche Weise generiert werden, beispielsweise über eine Peristaltikpumpe (Rollenpumpe) oder über eine Venturipumpe. Es ist darauf zu achten, dass die Geräuschentwicklung durch Betreiben der Pumpe so gering wie möglich ist, zumal sich die Einheit in unmittelbarer Nähe des Operateurs befindet.

Zu der den Unterdruck bereitstellenden Unterdruckkammer sei angemerkt, dass deren Unterdruck durch die Aspirationskammer hindurch wirkt, so dass nach Freigabe des Unterdrucks, d.h. nach Herstellen einer Strömungsverbindung durch die Aspirationskammer hindurch, die Flüssigkeit in die Aspirationskammer gesaugt wird. Geeignete Abdichtmaßnahmen bzw. Barrieren für die Flüssigkeit sind vorgesehen.

In ganz besonders vorteilhafter Weise wird der detektierte Füllstand in der Aspirationskammer zur Pumpenregelung, d.h. zur Erzeugung des Unterdrucks und/oder zum Abpumpen aus der Aspirationskammer herangezogen. Durch eine entsprechende Regelung kann erreicht werden, dass das Abpumpen der Aspirationflüssigkeit ab Erreichen einer bestimmten Marke mit geringer Pumpenleistung bereits beginnt oder dass man nach Abpumpen einer gewissen Menge die weitere Abführung der Aspirationsflüssigkeit mit geringer Pumpenleistung und somit geräuscharm generiert. So lässt sich mit einer "intelligenten" Regelung die Pumpenleistung reduzieren, beispielsweise durch frühzeitige Aktivierung der Pumpe, so dass diese dann mit wesentlich geringerer Leistung fortlaufend betrieben werden kann, nämlich dann, wenn die Aspirationskammer sukzessive abgepumpt wird. Die Geräuschentwicklung lässt sich dabei ganz erheblich reduzieren.

Die Kassette, die die Aspirationskammer und ggf. die Unterdruckkammer umfasst, ist in vorteilhafter Weise als Einschub-/Einsteckmodul zur Positionierung und zum Anschluss vorzugsweise innerhalb eines Einschub-/Einsteckschachts eines Gehäuses der Einheit ausgeführt. Somit ist es möglich, von außerhalb des Gehäuses den Einschub-/Einsteckschacht zu bedienen, nämlich das Einschub-/Einsteckmodul einzuschieben oder einzustecken und entsprechend zu entfernen bzw. auszutauschen.

In ganz besonders vorteilhafter Weise ist die Kassette und ist der Einschub-/Einsteckschacht derart ausgelegt und bestückt bzw. konzipiert, nämlich mit Anschlüssen ausgestattet, dass die Kassette durch das Einschieben/Einstecken komplett konnektiert und somit betriebsbereit ist. Eine einfache Handhabung der Kassette ist dadurch erreicht.

In weiter vorteilhafter Weise ist die Kassette zumindest teilweise oder bereichsweise durchsichtig ausgeführt, um nämlich durch die Wandung der Kassette hindurch den Füllstand zu sehen, insbesondere aber detektieren zu können. Im Rahmen einer solchen Ausgestaltung ist es von weiterem Vorteil, wenn die Füllstandsmessung lichtoptisch erfolgt, und zwar von außerhalb der Kassette. Die Sensorik ist dabei außerhalb der Kassette angeordnet bzw. vorgesehen, und zwar in dem die Kassette aufnehmenden Einschub-/Einsteckschacht in der Einheit, nämlich dahingehend, dass eine Detektion des Füllstands nach Einsetzen der Kassette automatisch erfolgen kann, ohne dass dazu weitere Vorkehrungen zu treffen sind.

Die Sensorik kann fest innerhalb des Einschub-/Einsteckschachts angeordnet sein. Ebenso kann diese als austauschbares Sensorikmoduls vorgesehen sein.

In weiter vorteilhafter Weise umfasst die Sensorik Dioden zum Aussenden von Licht und Fototransistoren zur Detektion des Lichts, jeweils in unterschiedlicher Höhe. Im Konkreten kann die Sensorik einerseits ein Diodenarray und andererseits ein Fototransistorarray umfassen, wobei die Arrays vorzugsweise vertikal angeordnet sind, nämlich zur Detektion des Füllstands.

Die zuvor genannte Sensorik ist in vorteilhafter Weise in einem Eckbereich der Kassette angeordnet, und misst durch die durchsichtige Wandung der Kassette hindurch. Zum Einkoppeln des Lichts in die Aspirationskammer und zum Auskoppeln des Lichts aus der Aspirationskammer ist in vorteilhafter Weise ein Prisma bzw. eine Prismaanordnung vorgesehen, so dass die Dioden und Fototransistoren die unter einem Winkel von 90° zueinander angeordnet sein können, das Licht über das Prisma ein- und auskoppeln, und zwar in Abhängigkeit vom Füllstand der Aspirationskammer. Eine eindeutige Füllstandsermittlung ist möglich, wobei die Signale über einen Mikrocontroller bzw. einen Prozessor ausgewertet und ein den Füllstand betreffendes Signal zur Vakuumerzeugung bzw. Pumpenregelung genutzt wird.

In erster Linie dient die optische Sensorik zur Füllstandsermittlung innerhalb der Aspirationskammer. Ebenso ist es denkbar, dass eine der Dioden und einer der Fototransistoren zum Erkennen der Anwesenheit bzw. der ordnungsgemäßen Positionierung der Kassette im Einschub-/Einsteckschacht dient. Bevorzugt werden dazu die oberste Diode und der oberste Fototransistor verwendet, um nämlich die diesbezüglichen Funktionen der Einheit - nach Detektion eines Vorhandenseins und ordnungsgemäßen Einsatzes der Kassette, freigeben zu können.

Wie bereits zuvor erwähnt, wird die in die Aspirationskammer der Kassette gesaugte Aspirationsflüssigkeit entsprechend der Detektion des Füllstands abgepumpt, nämlich in vorteilhafter Weise in einen separaten Aspirationsbeutel, der unterhalb der Einheit positionierbar bzw. an einem Maschinengestellt, Ständer oder dgl. aufhängbar ist. Von dort aus kann die Flüssigkeit entsorgt werden.

Schließlich ist es von Vorteil, wenn der Betriebszustand der Kassette, insbesondere der Füllstand in der Aspirationskammer und der in der Druckkammer aufgebaute Unterdruck, ggf. auch die aktuelle Pumpenleistung, optisch angezeigt werden. Dazu könnte ein Display der Einheit dienen. Eine farbige Grafik könnte die Betriebssituation der Kassette bzw. den Betriebszustand der Kassette visualisieren.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht die grundsätzliche Anordnung von Steuer-/Versorgungseinheit und Handgerät, verbunden über eine Saugleitung,
- Fig. 2: in einem schematischen Blockdiagramm die grundsätzliche Funktionsweise der Steuer-/Versorgungseinheit in Bezug auf die Füllstandsüberwachung in der Aspirationskammer,
- Fig. 3: in einer schematischen Darstellung die Anordnung der Dioden/Fototransistoren unter Zwischenschaltung einer austauschbaren Prismaanordnung und
- Fig. 4: in einem schematischen Blockdiagramm die grundsätzliche Funktionsweise einer Steuer-/Versorgungseinheit mit den erfindungsgemäßen Merkmalen, nämlich in Bezug auf die Füllstandsüberwachung im Vorratsbehälter.

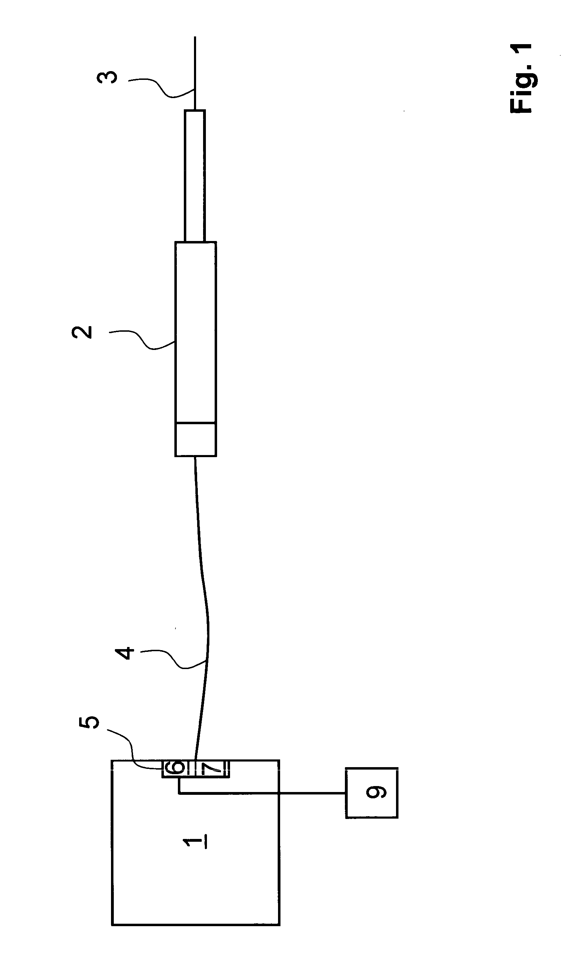

Fig. 1 zeigt schematisch die grundsätzliche Anordnung einer Steuer-/Versorgungseinheit 1 mit angeschlossenem Handgerät 2, wobei das Handgerät 2 eine Sauglanze 3 in Form einer Sonde umfasst, mit der Aspirationsflüssigkeit und Linsentrümmer bei einer Augenoperation aus dem Auge gesaugt werden. Sonstige Funktionen des Handgeräts 2 spielen hier keine Rolle, wobei es sich bei dem Handgerät 2 beispielsweise um einen sogenannten Cutter mit Versorgungsfunktion für Spülflüssigkeit und Absaugfunktion handeln kann. Lediglich die Absaugfunktion ist in Bezug auf die beanspruchte Lehre von Bedeutung.

Das Handgerät 2 ist über eine Saugleitung 4 mit der Steuer-/Versorgungseinheit 1 strömungsverbunden, wobei von Seiten der Steuer-/Versorgungseinheit 1 ein Unterdruck zum Absaugen der Aspirationsflüssigkeit zur Verfügung gestellt wird.

Innerhalb der Steuer-/Versorgungseinheit 1 ist eine besondere Kassette 5 vorgesehen, die eine Aspirationskammer 6 und eine Unterdruckkammer 7 umfasst.

Es ist eine Sensorik 8 zum Überwachen des Füllstands in der Aspirationskammer 6 vorgesehen, die bei Erreichen eines vorgegebenen Füllstands über ein elektrisches Signal das Abpumpen der in der Aspirationskammer 6 befindlichen Flüssigkeit in ein separates Behältnis 9 aktiviert.

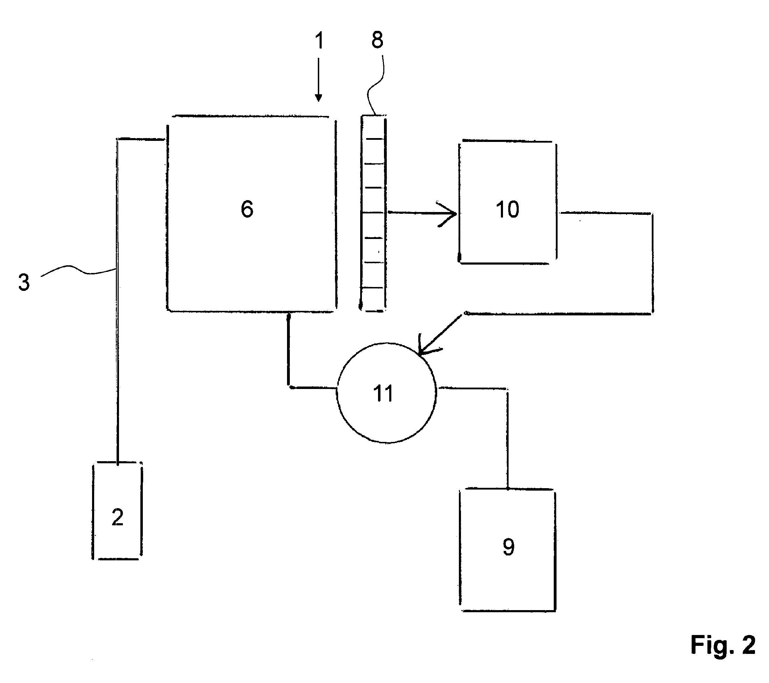

Fig. 2 zeigt den funktionalen Zusammenhang der hier relevanten Komponenten im Rahmen eines Blockschaltbildes, wobei auch dort zu erkennen ist, dass die Steuer-Nersorgungseinheit 1 über eine Saugleitung 3 mit dem Handgerät 2 strömungsverbunden ist. Die Aspirationskammer 6 wird über die Sensorik 8 in Form eines Diodenarrays/Fototransistorarrays überwacht, wobei die den jeweiligen Füllstand betreffenden Signale über einen Mikrocontroller 9 eine Pumpe 11 ansteuern bzw. den Pumpenbetrieb regeln. In einer in Fig. 2 nicht gezeigten Unterdruckkammer wird ein Unterdruck zum Ansaugen der Aspirationsflüssigkeit über das Handgerät 2 erzeugt. Gleichzeitig dient die Pumpe 11 zum vorzugsweise geregelten Abpumpen der Aspirationsflüssigkeit aus der Aspirationskammer 6 in einen tiefergelegenen Auffangbehälter 9.

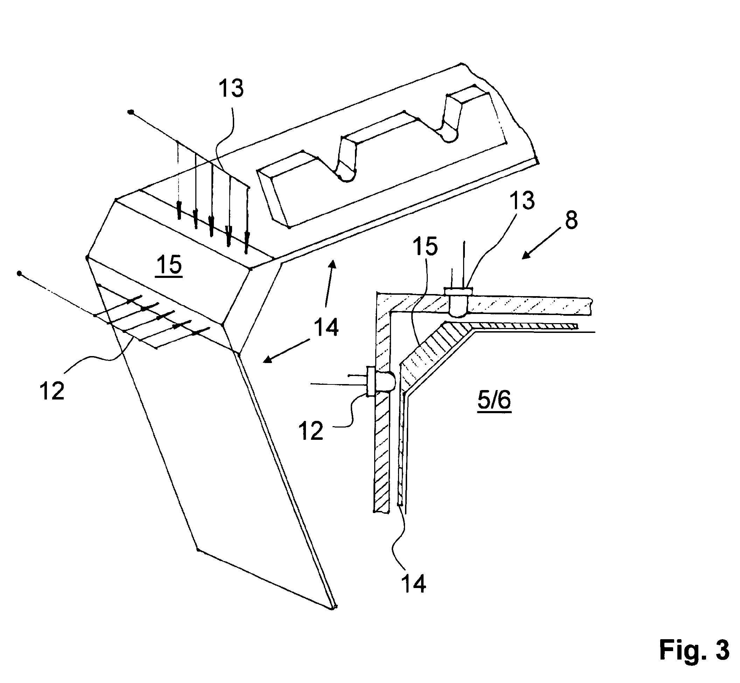

Fig. 3 zeigt ausschnittsweise und im Detail die Sensorik 8, die ein Diodenarray 12 und ein Transistorarray 13 zur an sich bekannten Detektion eines Füllstands umfasst. Die Kassette 5 bzw. die Aspirationskammer 6, in die die Aspirationsflüssigkeit gesaugt wird, ist in Fig. 3 lediglich angedeutet. Die Einkopplung und Auskopplung des Lichts erfolgt über eine zwischengeschaltete Prismaanordnung 14, die in den Einschub-/Einsteckschacht in der Einheit vor der dort fest angeordneten Sensorik 8 positionierbar ist. Eine Abstimmung auf die konkret verwendete Kassette 5 ist von Vorteil. Fig. 3 zeigt jedenfalls deutlich, dass die Prismaanordnung 14 ein im Eckbereich liegendes Prisma 15 umfasst, nämlich zum Ein- und Auskoppeln des Lichts.

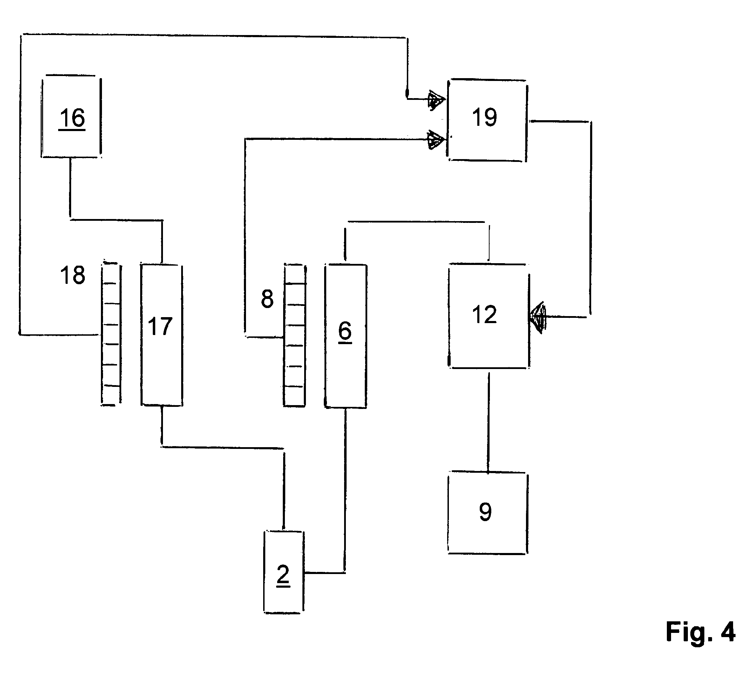

Fig. 4 zeigt eine erfindungsgemäße Steuer-/Versorgungseinheit, bei der eine Spülflüssigkeit oder dgl. über eine Infusion 16 bereitgestellt wird, nämlich in einem zur Aufnahme der Flüssigkeit dienenden Vorratsbehälter 17. Der Vorratsbehälter 17 ist in oder an der Steuer-/Versorgungseinheit 1 angeordnet und ist hinsichtlich des Füllstands bzw. der Füllstandsänderung über eine Sensorik 18 überwacht. Dabei kann es sich im Konkreten um die gleiche Sensorik handeln, wie die Sensorik 8 zur Überwachung der Aspirationskammer 6. Insoweit sei verwiesen auf die diesbezüglichen voranstehenden Ausführungen zu den Figuren 1 bis 3.

Die im Vorratsbehälter 17 befindliche Flüssigkeit wird zu dem Handgerät 2 gefördert und gelangt von dort aus - beispielsweise zum Spülen - in das menschliche Auge. Gleichermaßen wird die Flüssigkeit, als Aspirationsflüssigkeit, über das Handgerät 2 abgesaugt, nämlich in die Aspirationskammer 6, die in eine Kassette 5 entsprechend der Darstellung in Fig. 1 integriert sein kann. Auch hier wird der Füllstand überwacht bzw. detektiert, nämlich durch die Sensorik 8. Diese ist voranstehend unter Bezugnahme auf die Fig.1, 2 und 3 ausführlich beschrieben, so dass weitere Ausführungen dazu an dieser Stelle ebenfalls nicht erforderlich sind.

Elektrische Signale sowohl von der Sensorik 8 als auch von der Sensorik 18 werden zu einem Mikrocontroller 19 geleitet, der die erhaltenen Informationen verarbeitet, beispielsweise im Wege einer Differenzbildung, den Motor bzw. die Pumpe 11 ansteuert, wobei entsprechend dem detektierten Abfluss aus dem Vorratsbehälter 17 eine Regelung der Saugleistung zum Absaugen der Flüssigkeit aus der Aspirationskammer 6 heraus in den Auffangbehälter 9 erfolgt.

Voranstehende Ausführungen machen deutlich, dass die Pumpen-/Motorleistung der Pumpe 11 in Abhängigkeit vom Zufluss aus dem Vorratsbehälter 17 geregelt wird, wobei zur Bildung der Stellgröße Füllstandsänderungen sowohl in der Aspirationskammer 6 als auch im Vorratsbehälter 17 von Bedeutung sind.

Hinsichtlich weiterer Merkmale, die sich den Figuren nicht entnehmen lassen, sei zur Vermeidung von Wiederholungen auf den allgemeinen Teil der Beschreibung verwiesen.

Schließlich sei angemerkt, dass die voranstehend erörterten Ausführungsbeispiele lediglich der beispielhaften Erörterung der beanspruchten Lehre dienen, diese jedoch nicht auf die Ausführungsbeispiele einschränken.

### Bezugszeichenliste

- 1: Steuer-/Versorgungseinheit
- 2: Handgerät, Handstück
- 3: Sauglanze, Sonde
- 4: Saugleitung
- 5: Kassette
- 6: Aspirationskammer
- 7: Unterdruckkammer
- 8: Sensorik (für die Aspirationskammer)
- 9: Auffangbehälter, Behältnis
- 10: Mikrocontroller
- 11: Pumpe, Motor
- 12: Diodenarray
- 13: Fototransistorarray
- 14: Prismaanordnung
- 15: Prisma
- 16: Infusion
- 17: Vorratsbehälter
- 18: Sensorik (für den Vorratsbehälter)
- 19: Mikrocontroller

## Patentansprüche

1. Steuer-/Versorgungseinheit zum Betreiben mindestens eines medizinischen Handgeräts (2), Handstücks oder Handgriffs, insbesondere eines chirurgischen Handgeräts (2) zur Durchführung ophthalmologischer Operationen, wobei eine Versorgungsfunktion zum Versorgen eines Handgeräts mit einer Flüssigkeit aus einem Vorratsbehälter und eine Aspirationsfunktion zum Absaugen der Flüssigkeit über das Handgerät (2) in eine Aspirationskammer vorgesehen ist, in die die Flüssigkeit vom Handgerät (2) über einen Schlauch förderbar ist,
**dadurch gekennzeichnet, dass** eine Sensorik (8) zum Überwachen des Füllstands bzw. der Füllstandsänderung in dem Vorratsbehälter (17) und somit des Zuflusses pro Zeiteinheit zum Handgerät (2) vorgesehen ist und dass über die ermittelte Füllstandsänderung im Vorratsbehälter (17) bzw. über den ermittelten Zufluss zum Handgerät (2) die Pumpenleistung bzw. Saugleistung zum Absaugen der Flüssigkeit in die Aspirationskammer (6) regelbar ist.

2. Steuer-/Versorgungseinheit nach Anspruch 1, **dadurch gekennzeichnet, dass** die Füllstandsänderung über Gewichtssensoren, insbesondere über Wägezellen, oder über kapazitive oder lichtoptische Füllstandssensoren ermittelbar ist.

3. Steuer-/Versorgungseinheit nach Anspruch 1 oder 2, **dadurch gekennzeichnet, dass** die Pumpe (11) zu Beginn eines Absaugvorgangs mit hoher Spannung angesteuert und danach mit reduzierter Spannung, vorzugsweise gepulst, in Abhängigkeit der Füllstandsänderung im Vorratsbehälter (17) weiter betrieben wird.

4. Steuer-/Versorungseinheit nach einem der Ansprüche 1 bis 3, **dadurch gekennzeichnet, dass** bei Erreichen eines vorgegebenen Füllstands in der Aspirationskammer (6) über ein entsprechendes Signal das Abpumpen der in der Aspirationskammer (6) befindlichen Flüssigkeit in ein separates Behältnis (9) aktiviert wird, dass zum Absaugen der Flüssigkeit innerhalb der Kassette (5) Unterdruck erzeugt werden kann und wobei der Unterdruck vorzugsweise über eine Pumpe (11) erzeugbar ist.

5. Steuer-/Versorgungseinheit Anspruch 4, **dadurch gekennzeichnet, dass** der Unterdruck in einer separaten Unterdruckkammer (7) erzeugt wird, deren Unterdruck durch die Aspirationskammer (6) hindurch wirkt, so dass über den Unterdruck die Flüssigkeit in die Aspirationskammer (6) gesaugt wird.

6. Steuer-/Versorgungseinheit nach Anspruch 4 oder 5, **dadurch gekennzeichnet, dass** der detektierte Füllstand zur Pumpenregelung herangezogen wird.

7. Steuer-/Versorgungseinheit nach einem der Ansprüche 4 bis 6, **dadurch gekennzeichnet, dass** die Kassette (5) als Einschub-/Einsteckmodul zur Positionierung und zum Anschluss vorzugsweise innerhalb eines Einschub-/Einsteckschachts eines Gehäuses der Einheit ausgeführt ist, wobei die Kassette (5) und der Einschub-/Einsteckschacht derart ausgelegt und bestückt und mit Anschlüssen ausgestattet sein kann, dass die Kassette (5) durch das Einschieben/Einstecken betriebsbereit ist.

8. Steuer-/Versorgungseinheit nach einem der Ansprüche 4 bis 7, **dadurch gekennzeichnet, dass** die Kassette (5) zumindest teilweise oder bereichsweise durchsichtig ist.

9. Steuer-/Versorgungseinheit nach einem der Ansprüche 4 bis 8, **dadurch gekennzeichnet, dass** die Füllstandsmessung lichtoptisch erfolgt, wobei die Füllstandsmessung von außerhalb der Kassette (5) erfolgen kann und die Sensorik (8) außerhalb der Kassette (5), in dem die Kassette (5) aufnehmenden Einschub-/Einsteckschacht in der Einheit angeordnet ist.

10. Steuer-/Versorgungseinheit nach Anspruch 9, **dadurch gekennzeichnet, dass** die Sensorik (8) Dioden zum Aussenden von Licht und Fototransistoren zur Detektion des Lichts, jeweils in unterschiedlicher Höhe, aufweist.

11. Steuer-/Versorgungseinheit nach Anspruch 10, **dadurch gekennzeichnet, dass** die Sensorik (8) ein Diodenarray (12) und ein Fototransistorarray (13), vorzugsweise vertikal angeordnet, umfasst.

12. Steuer-/Versorgungseinheit nach Anspruch 10 oder 11, **dadurch gekennzeichnet, dass** zwischen den Dioden und den Fototransistoren ein Prisma (15) zum Einkoppeln des Lichts in die Aspirationskammer (6) und zum Auskoppeln des Lichts aus der Aspirationskammer (6) angeordnet ist.

13. Steuer-/Versorgungseinheit nach einem der Ansprüche 10 bis 12, **dadurch gekennzeichnet, dass** die oberste Diode zur Erkennung der Anwesenheit bzw. der ordnungsgemäßen Positionierung der Kassette (5) im Einschub-/Einsteckschacht umfasst.

14. Steuer-/Versorgungseinheit nach einem der Ansprüche 4 bis 13, **dadurch gekennzeichnet, dass** das Abpumpen der Flüssigkeit in einen separaten Aspirationsbeutel erfolgt.

15. Steuer-/Versorgungseinheit nach einem der Ansprüche 1 bis 14, **dadurch gekennzeichnet, dass** der Betriebszustand optisch angezeigt wird, vorzugsweise auf einem Display der Einheit.
